(19) Europäisches Patentamt
European Patent Office
Office européen des brevets

(11) Publication number: **0 322 738 B1**

# (12) EUROPEAN PATENT SPECIFICATION

(45) Date of publication of patent specification: **12.04.95**

(51) Int. Cl.⁶: **A61K 31/275**, C07C 255/36

(21) Application number: **88121405.0**

(22) Date of filing: **21.12.88**

Divisional application 93119976.4 filed on 21/12/88.

## (54) Benzylidene-malononitrile derivatives for the inhibition of proliferative processes in mammalian cells.

(30) Priority: **24.12.87 IL 84937**
**10.11.88 IL 88354**

(43) Date of publication of application:
**05.07.89 Bulletin 89/27**

(45) Publication of the grant of the patent:
**12.04.95 Bulletin 95/15**

(84) Designated Contracting States:
**AT BE CH DE ES FR GB GR IT LI LU NL SE**

(56) References cited:
**DE-A- 3 740 383**
**US-A- 3 522 188**
**US-A- 4 064 266**

**CHEM.PHARM. BULL., vol. 34, no. 4, 1986, pages 1619-1627, I. KATSUMI et al.**

**CHIMIE THERAPEUT., no. 2, March-April 1973, pages 188-193, J.-C. DORE et al.**

**PESTICIDES, vol. 18, no. 2, February 1984, pages 30, 36, M.R. MANRO et al**

(73) Proprietor: **YISSUM RESEARCH DEVELOPMENT COMPANY OF THE HEBREW UNIVERSITY OF JERUSALEM**
**46 Jabotinsky Street**
**Jerusalem, 92 182 (IL)**

(72) Inventor: **Levitzki, Alexander**
**36 Palmach Street**
**Jerusalem (IL)**
Inventor: **Gilon, Chaim**
**18 Gelbert Street**
**Jerusalem (IL)**
Inventor: **Chorev, Michael**
**35 Feinstein Street**
**Talpiot Mizrach, Jerusalem (IL)**
Inventor: **Gazit, Aviv**
**14 Nof Harim Street**
**Jerusalem (IL)**

(74) Representative: **Brown, John David et al**
**FORRESTER & BOEHMERT**
**Franz-Joseph-Strasse 38**
**D-80801 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 322 738 B1

**BR. J. PHARMACOL.**, vol. 44, 1972 pages 561-576, **R.W. BRIMBLECOMBE** et al.

**PATENT ABSTRACTS OF JAPAN**, vol. 11, no. 222 (C-435)(2669), July 18. 1987

**LIEBIGS ANN.**, vol. 437, 1924, pages 125-147, **K.W. ROSENMUND** et al.

## Description

### FIELD OF INVENTION

The present invention concerns novel pharmaceutical compositions containing substituted benzylidene- and cinnamylidene-malononic acid derivatives for specifically inhibiting cell proliferation processes in mammals. The invention further provides certain novel compounds of the aforesaid type.

### BACKGROUND OF THE INVENTION

Currently the chemotherapy of cancer makes use of inhibitors of DNA synthesis (examples: adriamycin, fluorouracil) and compounds which disrupt the cytoskeleton (vinblastine). These compounds are highly toxic since their inhibitory activity is not limited to cancer cells, with the distinction, however, that tumor cells are more readily attacked by the aforesaid inhibitors because these cells divide more rapidly and their DNA metabolism is consequently more active. A few types of cancers are nowadays treated with specific pharmaceutical agents. These include, for example, certain breast cancers which are hormone dependent and can therefore be treated with specific hormone derivatives. These cases, however, are the exception and the chemotherapeutic treatment for the majority of the various types of cancer is non-specific.

In the early 1980's it became apparent that 20 to 30 percent of cancers express characteristic oncogenic products which are growth factor receptors or their mutated homologs, which exhibit protein tyrosine kinase (PTK) activity. The PTK activity is intrinsic to the receptor or its oncogene homolog and, which influences the cell proliferation via its PTK domain. Furthermore, each of these receptors (normal or mutated), exhibits a characteristic PTK activity with a distinct substrate specificity. One of these receptors is the epidermal growth factor (EGF) receptor and its oncogenic homolog v-Erb-b. Pursuant to that discovery it has already been proposed to treat cancer by means of various chemical substances capable of inhibiting the PTK activity of EGF - see, for example, in Japanese patents Nos. 6239523, 6242923 and 6242925.

It is the object of the present invention to provide readily accessible compounds of relatively simple structure that are active as specific EGFR-tyrosine kinase inhibitors and can thus serve as specific anti-cancer agents.

### GENERAL DESCRIPTION OF THE INVENTION

The above object is achieved by the present invention which provides pharmaceutical composition for inhibiting cell proliferation processes, which composition comprises, as an active ingredient, in combination with a pharmaceutically acceptable carrier, a compound of the general formula:

(A) an α-substituted benzylidene malononitrile compound having the formula:

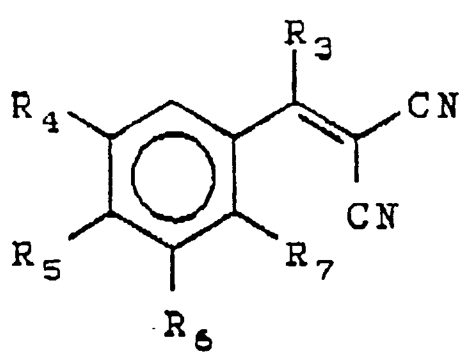

Ia

wherein:

$R_3$ is $CH_3$ or OH;

$R_4$, $R_5$, $R_6$ and $R_7$ are each independently alkyl of 1-5 carbons, H, halogen, OR, CHO, COOH, $NH_2$ or $NO_2$; and R is alkyl of 1-5 carbons or H; or

(B) an α-unsubstituted benzylidene malononitrile compound having the formula:

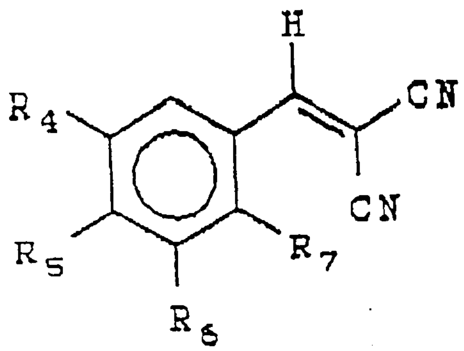

Ib

wherein:

at least one of $R_4$, $R_5$, $R_6$ and $R_7$ is alkyl of 2-5 carbons, CHO, COOH or $NH_2$;

the remainder of $R_4$, $R_5$, $R_6$ and $R_7$ are each independently alkyl of 1-5 carbons, H, halogen, OR, CHO, COOH, $NH_2$ or $NO_2$;

and R is alkyl of 1-5 carbons or H;

provided that, when either of $R_4$ and $R_6$ is t-butyl or t-amyl and $R_7$ is H, halogen or alkyl of 1-5 carbons then $R_5$ is not OH; and when each of $R_4$ and $R_5$ is OR and R is hydrogen or alkyl of 2-5 carbons, then $R_6$ and $R_7$ are independently not halogen, $NO_2$, CHO or COOH;

or a pharmaceutically acceptable salt thereof, as well as the use of such α-substituted benzylidene malononitrile compounds of formula Ia or Ib for the preparation of a medicament which inhibits cell proliferation processes.

Preferred active ingredients are compounds of formula Ib wherein $R_4$ and $R_5$ are independently OH, $R_6$ is OH and $R_3$ and $R_7$ are independently hydrogen, or a pharmaceutically acceptable salt thereof, as well as a compound selected from:

α-hydroxy-(3,4,5-trihydroxybenzylidene) - malononitrile; and 4-formylbenzylidene-malononitrile;

and pharmaceutically acceptable salts thereof.

The malonic acid derivatives of formula (I) above, can be prepared by known methods, for example by reacting a corresponding substituted benzaldehyde with malononitrile to obtain the benzylidene derivatives or with malononitrile dimers to obtain the cinnamylidene derivatives. The reaction is generally carried out in a suitable solvent, such as ethanol or benzene, and in the presence of a catalyst, e.g., piperidine, pyridine or β-alanine. Alternatively, a suitably substituted benzoyl chloride, e.g. triacetyl-galloyl chloride, can be reacted with malononitrile in the presence of an amine in a non-polar organic solvent.

The EGFR-inhibitor activity was tested on partially purified EGF receptors and on cell culture samples and the results are summarized in Table 1 herein.

The invention will now be described in more detail in the following non-limiting examples. Only Example 2 is in accordance with the present invention. The other Examples are to assist the person skilled in the art in performing the invention

## Preparative Examples

### Example 1

3,4-Dihydroxybenzylidene-malononitrile
(Table 1, Compound 2)

To 11g (80 mM) of 3,4-dihydroxybenzaldehyde and 5.5g (83 mM) of malononitrile in 40 ml of ethanol, 7 drops of piperidine were added. The mixture was then heated at 70 °C for 0.5-1 hour and then poured into water. The resulting solid precipitate was separated by filtration to give 12.7g (86% yield) of a yellow solid, 3,4-dihydroxybenzylidene-malononitrile, m.p.225.

Following the same procedure there were prepared:

3,5-dihydroxybenzylidene-malononitrile (Compound 1 in Table 1),

3-methoxy-4,5-dihydroxybenzylidene-malononitrile (Compound 3 in Table 1),

3,4,5-trihydroxybenzylidene-malononitrile (Compound 4 in Table 1).
3,5-di-t-butyl-4-hydroxybenzylidene-malononitrile,
3-hydroxybenzylidene-malononitrile.

**Example 2**

α-hydroxy-3,4,5-trihydroxybenzylidene-malononitrile
(Table 1, Compound 5)

To 2g (30mM) of malononitrile and 4 ml (40 mM) of triethylamine in 100 ml of $CH_2Cl_2$, triacetyl galloyl chloride (prepared from 7g (24 mM) of triacetyl gallic acid and thionyl chloride) in 50 ml $CH_2Cl_2$ was added. The resulting mixture was then stirred for 2 hours at room temperature, poured into 50 ml of water and hydrolyzed by heating for 2 minutes at 80 °C with a solution of 2.5g of NaOH in 30 ml of ethanol. The mixture was then extracted with ethyl acetate and the organic extract was further worked up by washing with water, drying, filtering and evaporating it. Chromatography on silica gel gave 1.5g (29% yield) of α-hydroxy 3,4,5-trihydroxybenzylidene-malononitrile as an oily solid.

**Example 3**

α-cyano-3,4-dihydroxycinnamamide
(Table 1, Compound 6)

Reaction of 2.4g (10 mM) of 3,4-dihydroxybenzaldehyde and 0.9g (10.7 mM) of cyanoacetamide, by the procedure described in Example 1 above, gave 1.45g (70% yield) of α-cyano-3,4-dihydroxycinnamamide, as a yellow solid, m.p.247 °C.

**Example 4**

γ-cyano-β-amino-3,4-dihydroxycinnamylidene-malononitrile
(Table 1, Compound 7)

1.4g (10 mM) of 3,4-dihydroxybenzaldehyde, 1.4g (10.6 mM) of malononitrile dimer and 0.3g of β-alanine in 50 ml ethanol were heated at 70 °C for 40 minutes. 100 ml of water were added and the suspension was cooled and a solid precipitate was filtered off, washed with water and dried to give 1.3g of a yellow-orange solid, mp.235 °C, 53% yield, of γ-cyano-β-amino-3,4-dihydroxycinnamylidene-malononitrile.

Following the same procedure there were prepared:
γ-Cyano-β-amino-3,4,5-trihydroxycinnamylidene-malononitrile (Compound 12 in Table 1), and
γ-Cyano-β-amino-3-hydroxy-4-nitrocinnamylidene-malononitrile (Compound 15 in Table 1).

**Example 5**

α-cyano-3,4-dihydroxycinnamic acid
(Table 1, Compound 8)

a) 2g (15 mM) of 3,4-dihydroxybenzaldehyde, 3g (21 mM) of t-butyl cyanoacetate and 0.5 ml of piperidine in 50 ml ethanol were heated to reflux for 1 hour. The resulting mixture was then poured into water and a solid precipitate was separated by filtration and was then washed and dried to yield 2.5g (yield 66%) of t-butyl α-cyano-3,4-dihydroxycinnamate as a yellow solid.

b) 1.6g of the t-butyl ester from a) in 10 ml of Trifluoro Acetic Acid was stirred at room temperature for 20 minutes. 50 ml of $H_2O$ were added and the cooled suspension filtered to yield a solid precipitate which was washed with water and dried to give 1g (yield 85%) of α-cyano-3,4-dihydroxycinnamic acid as a yellow solid, mp.240 °C.

**Example 6**

α-cyano-3,4-dihydroxycinnamthioamide
(Table 1, Compound 9)

To 0.83g (6 mM) 3,4-dihydroxybenzaldehyde and 0.7g (7 mM) cyanothioacetamide in 30 ml ethanol were added 4 drops of piperidine. The mixture was refluxed for 1 hour and poured into ice-water. Filtering and drying gave 0.54 g, (41% yield), of an orange solid, mp.213 °C.

| | | | |
|---|---|---|---|
| Anal. Calc. for $C_{10}H_8N_2O_2S$: | C = 54.54, | H = 3.64, | N = 12.73; |
| Found: | C = 54.44, | H = 3.87, | N = 12.91 |

MS: 220(M+,93%), 219(M-1,100%), 203(M-OH,26%), 186(M-2OH,24%), 123(33%), 110(30%), 100-(43%), m/e.

**Example 7**

3,4-methylenedioxy-6-nitrobenzylidene malononitrile
(Table 1, Compound 11)

1g (5.1 mM) 3,4-methylenedioxy-6-nitrobenzaldehyde, 0.4 g (6 mM) malononitrile and 0.2 g β-alanine in 30 ml ethanol were stirred 16 hours at room temperature. 50 ml $H_20$ were added. Filtering gave 1g, (80% yield) of a bright yellow solid, mp.104 °C.
NMR (acetone-$d_{6)}$: 6.42 (2H, s, methylenedioxy), 7.45 (1H, s, $H_2$), 7.82 (1H, s, $H_5$), 8.70 (1H, s, vinylic proton).

Following the same procedure there were also prepared:
γ-Cyano-β-amino-3,4-dihydroxy-5-methoxycinnamylidene-malononitrile (Compound 13 in Table 1), and
γ-Cyano-β-amino-3,4-dihydroxy-5-bromocinnamylidene-malononitrile (Compound 14 in Table 1).

In Table 1 below there are listed 15 compounds, according to the invention, All 5 and 10 of which are compounds gave correct analytical and spectroscopic data.

In this Table, KInh is the dissociation constant of the complex PTK-inhibitor and is expressed in μM units. The different KInh values were determined by the analysis according to Dixon.

**Table 1**

| No. | | mp, °C | KInh, µM | Literature |
|---|---|---|---|---|
| 1 | 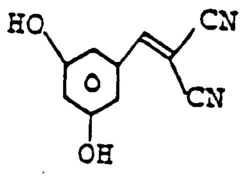 | 175 | 10 | New |
| 2 | 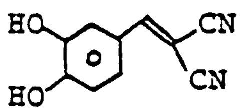 | 225 | 11±0.1 | 1* |
| 3 | 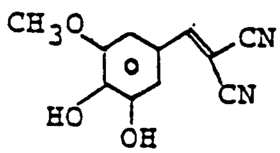 | 235 | 2.2±0.3 | New |
| 4 | 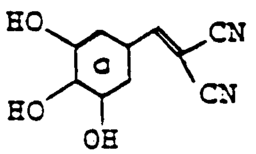 | 245 | 1 | 1* |
| 5 | 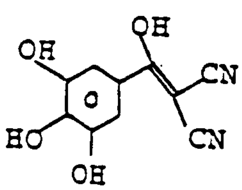 | oil | 4.5 | New |
| 6 | 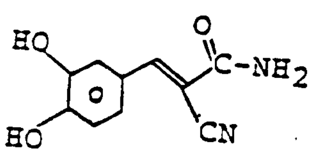 | 247 | 3.5±0.6 | 1* |
| 7 | 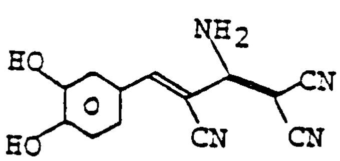 | 235 | Non-competitive inhibitor | New |

<u>**Table 1 (continued)**</u>

| No. | | mp, °C | KInh, μM | Literature |
|---|---|---|---|---|
| 8 | 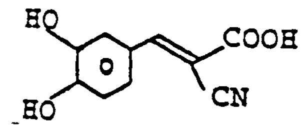 | 240 | 23.6 | 1* |
| 9. | 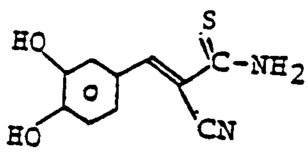 | 213 | 0.85 | New |
| 10. | 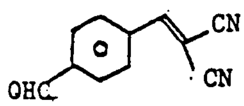 | 142 | 20 | New |
| 11. | 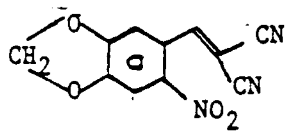 | 104 | - ** | New |
| 12. | 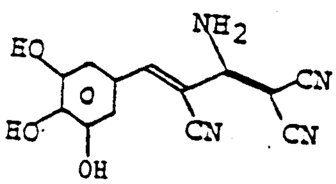 | 275 | Non-competitive inhibitor | New |
| 13. | 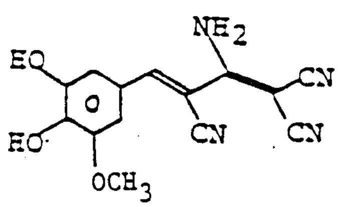 | 225 | Non-competitive inhibitor | New |
| 14. | 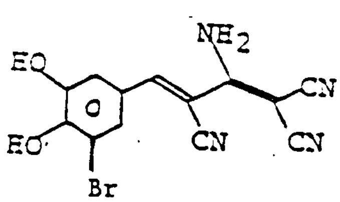 | 241 | Non-competitive inhibitor | New |

Table 1 (continued)

| No. | | mp, °C | KInh, μM | Literature |
|---|---|---|---|---|
| 15. | 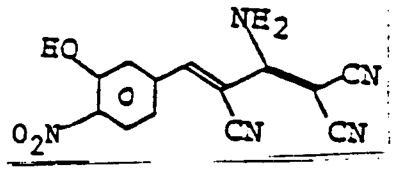 | 219 | Non-competitive inhibitor | New |

* K.W. Rosemund and T. Boehm, ann. 437, 125 (1924).
** KInh was not calculated.

EGFR Inhibition Tests

Test on extracted EGF Receptors:

EGF receptors were prepared from A431 cells (obtained from the ATCC) and PTK activity of these receptors was assayed as described by S. Braun, W. E. Raymond and E. Racker, J. Biol. Chem. 259, 2051-2054 (1984). The compounds listed in Table 1 were tested for their inhibitory capacity on the EGF-receptor kinase activity, using the assay described above. Dissociation constants were calculated from the inhibition curves and are listed in Table 1.

Tests on cells in tissue culture:

a) A431 cells and KB cells express EGF receptors on their cell surface and their growth rate depends on the presence of growth factors in the medium.
These cells were seeded and grown as described in O.Kashles and A.Levitzki, Bichem. Pharmacol., 35, 1531-1536 (1987). The inhibitor was added to the medium 1 hour after seeding. The medium volume in a well was 1 ml and the concentration of inhibitor therein 20 μM. Every 24 hours cells were counted and fresh medium with inhibitor applied to the remaining wells. The growth curves were determined in 24-well Costar dishes.
b) Some of the compounds according to the present invention are exclusive inhibitors to EGF dependent growth of cells and others are preferential inhibitors to such growth.

## Claims

**1.** A pharmaceutical composition for inhibiting cell proliferation processes, which composition comprises, as an active ingredient, in combination with a pharmaceutically acceptable carrier, a compound of the general formula:

(A) an α-substituted benzylidene malononitrile compound having the formula:

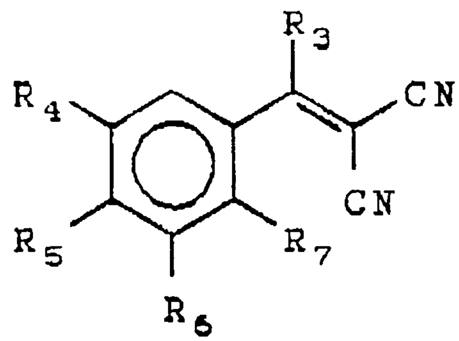

Ia

wherein:
$R_3$ is $CH_3$ or OH;
$R_4$, $R_5$, $R_6$ and $R_7$ are each independently alkyl of 1-5 carbons, H, halogen, OR, CHO, COOH, $NH_2$ or $NO_2$; and R is alkyl of 1-5 carbons or H; or
(B) an α-unsubstituted benzylidene malononitrile compound having the formula:

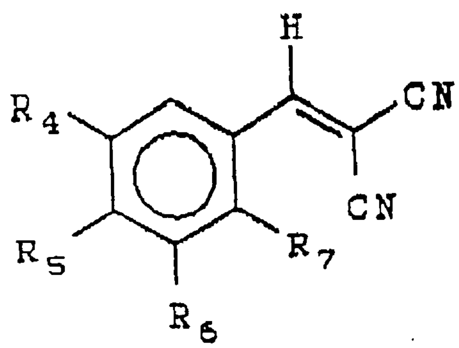

Ib

wherein:
at least one of $R_4$, $R_5$, $R_6$ and $R_7$ is alkyl of 2-5 carbons, CHO, COOH or $NH_2$;
the remainder of $R_4$, $R_5$, $R_6$ and $R_7$ are each independently alkyl of 1-5 carbons, H, halogen, OR, CHO, COOH, $NH_2$ or $NO_2$;
and R is alkyl of 1-5 carbons or H;
provided that, when either of $R_4$ and $R_6$ is t-butyl or t-amyl and $R_7$ is H, halogen or alkyl of 1-5 carbons then $R_5$ is not OH; and when each of $R_4$ and $R_5$ is OR and R is hydrogen or alkyl of 2-5 carbons, then $R_6$ and $R_7$ are independently not halogen, $NO_2$, CHO or COOH;
or a pharmaceutically acceptable salt thereof.

**2.** A pharmaceutical composition according to Claim 1, which comprises, as an active ingredient, a compound of formula Ib wherein $R_4$ and $R_5$ are independently OH, $R_6$ is OH and $R_3$ and $R_7$ are independently hydrogen, or a pharmaceutically acceptable salt thereof.

**3.** A pharmaceutical composition according to Claim 1, which comprises, as an active ingredient, a compound selected from:
α-hydroxy-(3,4,5-trihydroxybenzylidene)-malononitrile; and 4-formylbenzylidene-malononitrile;
and pharmaceutically acceptable salts thereof.

**4.** The use of a compound of the general formula:

(A) an α-substituted benzylidene malononitrile compound having the formula:

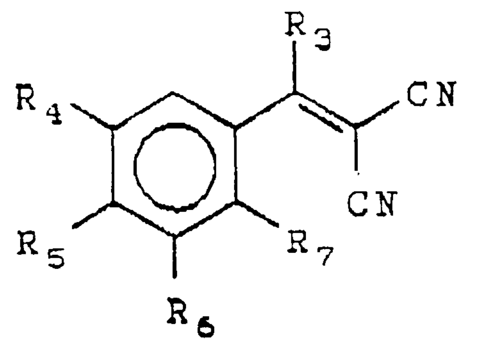

Ia

wherein:
$R_3$ is $CH_3$ or OH;
$R_4$, $R_5$, $R_6$ and $R_7$ are each independently alkyl of 1-5 carbons, H, halogen, OR, CHO, COOH, $NH_2$ or $NO_2$; and R is alkyl of 1-5 carbons or H; or
(B) an α-unsubstituted benzylidene malononitrile compound having the formula:

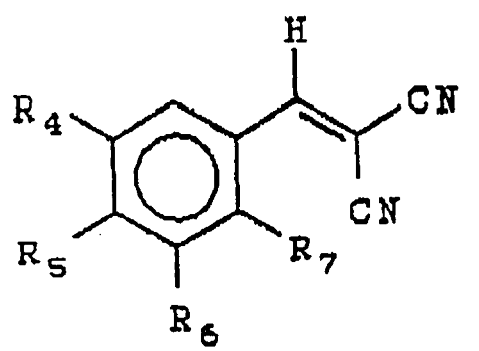

Ib

wherein:
at least one of $R_4$, $R_5$, $R_6$ and $R_7$ is alkyl of 2-5 carbons, CHO, COOH or $NH_2$;
the remainder of $R_4$, $R_5$, $R_6$ and $R_7$ are each independently alkyl of 1-5 carbons, H, halogen, OR, CHO, COOH, $NH_2$ or $NO_2$;
and R is alkyl of 1-5 carbons or H;
provided that, when either of $R_4$ and $R_6$ is t-butyl or t-amyl and $R_7$ is H, halogen or alkyl of 1-5 carbons then $R_5$ is not OH; and when each of $R_4$ and $R_5$ is OR and R is hydrogen or alkyl of 2-5 carbons, then $R_6$ and $R_7$ are independently not halogen, $NO_2$, CHO or COOH;

or a pharmaceutically acceptable salt thereof, for the preparation of a medicament which inhibits cell proliferation processes.

**5.** The use according to Claim 4, wherein the compound is of formula Ib, wherein $R_4$ and $R_5$ are independently OH, $R_6$ is OH and $R_3$ and $R_7$ are independently hydrogen, or a pharmaceutically acceptable salt thereof.

**6.** The use according to Claim 4, wherein the compound is selected from:
α-hydroxy-(3,4,5-trihydroxybenzylidene)-malononitrile; and 4-formylbenzylidene-malononitrile;
and pharmaceutically acceptable salts thereof.

## Patentansprüche

**1.** Eine pharmazeutische Zusammensetzung zur Hemmung von Zellproliferationsprozessen, wobei die Zusammensetzung, als einen aktiven Wirkstoff, in Kombination mit einem pharmazeutisch annehmbaren Trägerstoff, eine Verbindung der allgemeinen Formel:

A) eine α-substituierte Benzylidenmalononitril-Verbindung mit der Formel:

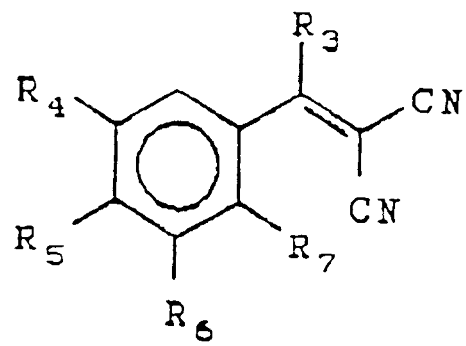

worin:

$R_3$ $CH_3$ oder OH ist;

$R_4$, $R_5$, $R_6$ und $R_7$ unabhängig voneinander jeweils Alkyl mit 1-5 Kohlenstoffen, H, Halogen, OR, CHO, COOH, $NH_2$ oder $NO_2$ sind;

und R Alkyl mit 1-5 Kohlenstoffen oder H ist; oder

B) eine α-unsubstituierte Benzylidenmalononitril-Verbindung mit der Formel:

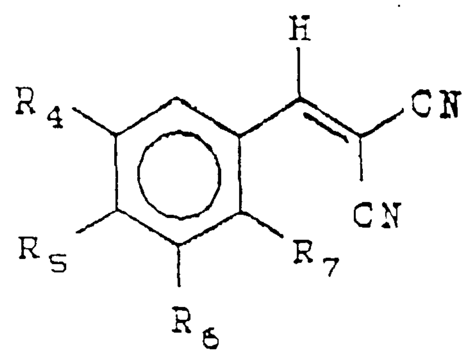

worin:

wenigstens eine der Gruppen $R_4$, $R_5$, $R_6$ und $R_7$ Alkyl mit 2-5 Kohlenstoffen, CHO, COOH oder $NH_2$ ist;

die restlichen Gruppen aus $R_4$, $R_5$, $R_6$ und $R_7$ unabhängig voneinander jeweils Alkyl mit 1-5 Kohlenstoffen, H, Halogen, OR, CHO, COOH, $NH_2$ oder $NO_2$ sind; und R Alkyl mit 1-5 Kohlenstoffen oder H ist;

vorausgesetzt, daß, wenn eine der Gruppen $R_4$ und $R_6$ tert.-Butyl oder tert.-Amyl ist und $R_7$ H, Halogen oder Alkyl mit 1-5 Kohlenstoffen ist, $R_5$ dann nicht OH ist; und wenn jede der Gruppen $R_4$ und $R_5$ OR ist und R Wasserstoff oder Alkyl mit 2-5 Kohlenstoffen ist, $R_6$ und $R_7$ dann unabhängig voneinander nicht Halogen, $NO_2$, CHO oder COOH sind;

oder ein pharmazeutisch annehmbares Salz derselben umfaßt.

**2.** Eine pharmazeutische Zusammensetzung nach Anspruch 1, die, als einen Wirkstoff, eine Verbindung von Formel Ib, worin $R_4$ und $R_5$ unabhängig voneinander OH sind, $R_6$ OH ist und $R_3$ und $R_7$ unabhängig voneinander Wasserstoff sind, oder ein pharmazeutisch annehmbares Salz derselben umfaßt.

**3.** Eine pharmazeutische Zusammensetzung nach Anspruch 1, die, als einen Wirkstoff, eine Verbindung umfaßt, die ausgewählt ist aus:

α-Hydroxy-(3,4,5-trihydroxybenzyliden)malononitril; und 4-Formylbenzylidenmalononitril;

und pharmazeutisch annehmbaren Salzen derselben.

**4.** Die Verwendung einer Verbindung der allgemeinen Formel:
(A) eine α-substituierte Benzylidenmalononitril-Verbindung mit der Formel:

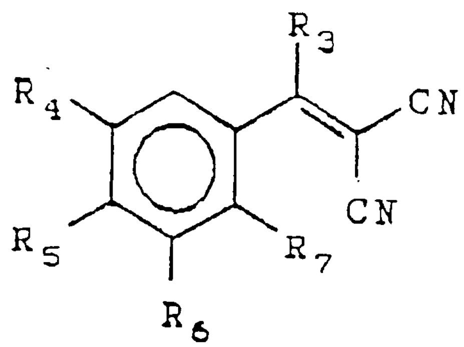

worin:
$R_3$ $CH_3$ oder OH ist;
$R_4$, $R_5$, $R_6$ oder $R_7$ unabhängig voneinander jeweils Alkyl mit 1-5 Kohlenstoffen, H, Halogen, OR, CHO, COOH, $NH_2$ oder $NO_2$ sind;
und R Alkyl mit 1-5 Kohlenstoffen oder H ist; oder
(B) eine α-unsubstituierte Benzylidenmalononitril-Verbindung mit der Formel:

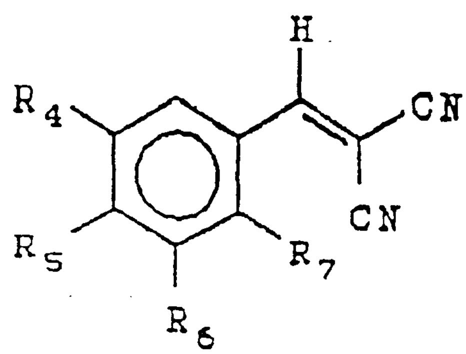

worin:
wenigstens eine der Gruppen $R_4$, $R_5$, $R_6$ und $R_7$ Alkyl mit 2-5 Kohlenstoffen, CHO, COOH oder $NH_2$ ist;
die restlichen Gruppen aus $R_4$, $R_5$, $R_6$ und $R_7$ unabhängig voneinander jeweils Alkyl mit 1-5 Kohlenstoffen, H, Halogen, OR, CHO, COOH, $NH_2$ oder $NO_2$ sind; und R Alkyl mit 1-5 Kohlenstoffen oder H ist;
vorausgesetzt, daß, wenn eine der Gruppen $R_4$ und $R_6$ tert.-Butyl oder tert.-Amyl ist $R_7$ H, Halogen oder Alkyl mit 1-5 Kohlenstoffen ist, $R_5$ dann nicht OH ist; und wenn jede der Gruppen $R_4$ und $R_5$ OR ist und R Wasserstoff oder Alkyl mit 2-5 Kohlenstoffen ist, $R_6$ und $R_7$ dann unabhängig voneinander nicht Halogen, $NO_2$, CHO oder COOH sind;
oder eines pharmazeutisch annehmbaren Salzes derselben zur Herstellung eines Arzneimittels, das Zellproliferationsprozesse hemmt.

**5.** Die Verwendung nach Anspruch 4, wobei die Verbindung die Formel Ib besitzt, worin $R_4$ und $R_5$ unabhängig voneinander OH sind, $R_6$ OH ist und $R_3$ und $R_7$ unabhängig voneinander Wasserstoff sind, oder ein pharmazeutisch annehmbares Salz derselben ist.

**6.** Verwendung nach Anspruch 4, wobei die Verbindung ausgewählt ist aus:
α-Hydroxy-(3,4,5-trihydroxybenzyliden)malononitril; und 4-Formylbenzylidenmalononitril;
und pharmazeutisch annehmbaren Salzen derselben.

## Revendications

**1.** Composition pharmaceutique pour inhiber les processus de prolifération cellulaire, ladite composition comprenant comme ingrédient actif, en combinaison avec un support acceptable en pharmacie, un composé consistant en :

(A) un (benzylidène α-substitué)-malononitrile de formule générale :

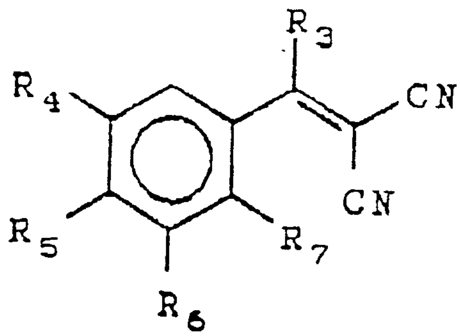

Ia

dans laquelle :
$R_3$ est $CH_3$ ou OH ;
$R_4$, $R_5$, $R_6$ et $R_7$ sont chacun indépendamment un alkyle en $C_1$-$C_5$, H, un halogène, OR, CHO, COOH, $NH_2$ ou $NO_2$; et R est un alkyle en $C_1$-$C_5$ ou H; ou

(B) un (benzylidène non α-substitué)-malononitrile de formule générale :

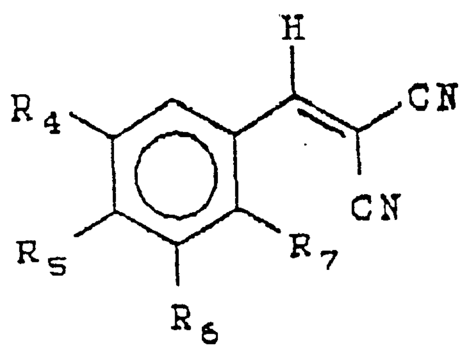

Ib

dans laquelle
l'un au moins des restes $R_4$, $R_5$, $R_6$ et $R_7$ est un alkyle en $C_2$-$C_5$, CHO, COOH ou $NH_2$ ;
les autres des restes $R_4$, $R_5$, $R_6$ et $R_7$ sont chacun indépendamment un alkyle en $C_1$-$C_5$, H, un halogène, OR, CHO, COOH, $NH_2$ ou $NO_2$ ; et R est un alkyle en $C_1$-$C_5$ ou H ; pourvu que lorsque l'un ou l'autre des restes $R_4$ et $R_6$ est t-butyle ou t-amyle et $R_7$ est H, un halogène ou un alkyle en $C_1$-$C_5$, alors $R_5$ ne soit pas OH ; et que lorsque $R_4$ et $R_5$ sont chacun OR et R est l'hydrogène ou un alkyle en $C_2$-$C_5$, alors $R_6$ et $R_7$ ne soient pas chacun indépendamment un halogène, $NO_2$, CHO ou COOH ;

ou un de ses sels acceptables en pharmacie.

**2.** Composition pharmaceutique selon la revendication 1, qui comprend, comme ingrédient actif, un composé de formule Ib dans laquelle $R_4$ et $R_5$ sont indépendamment OH, $R_6$ est OH et $R_3$ et $R_7$ sont indépendamment l'hydrogène, ou un de ses sels acceptables en pharmacie.

**3.** Composition pharmaceutique selon la revendication 1, qui comprend comme ingrédient actif, un composé choisi parmi l'α-hydroxy-(3,4,5-trihydroxybenzylidène)malononitrile, le 4-formylbenzylidène-malononitrile et leurs sels acceptables en pharmacie.

**4.** Utilisation d'un composé consistant en :

(A) un (benzylidène α-substitué)-malononitrile de formule générale :

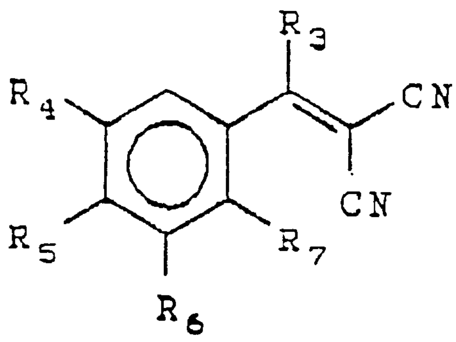

Ia

dans laquelle :

$R_3$ est $CH_3$ ou OH ;

$R_4$, $R_5$, $R_6$ et $R_7$ sont chacun indépendamment un alkyle en $C_1$-$C_5$, H, un halogène, OR, CHO, COOH, $NH_2$ ou $NO_2$ ; et R est un alkyle en $C_1$-$C_5$ ou H ; ou

(B) un (benzylidène non α-substitué)-malononitrile de formule générale :

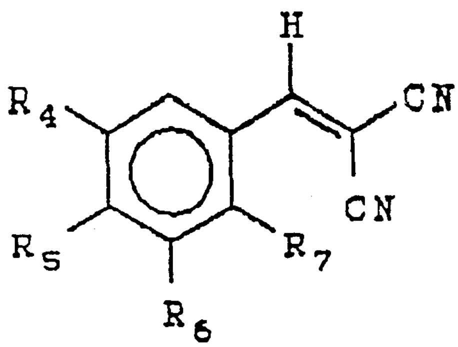

Ib

dans laquelle

l'un au moins des restes $R_4$, $R_5$, $R_6$ et $R_7$ est un alkyle en $C_2$-$C_5$, CHO, COOH ou $NH_2$ ;

les autres des restes $R_4$, $R_5$, $R_6$ et $R_7$ sont chacun indépendamment un alkyle en $C_1$-$C_5$, H, un halogène, OR, CHO, COOH, $NH_2$ ou $NO_2$ ; et R est un alkyle en $C_1$-$C_5$ ou H ; pourvu que lorsque l'un ou l'autre des restes $R_4$ et $R_6$ est t-butyle ou t-amyle et $R_7$ est H, un halogène ou un alkyle en $C_1$-$C_5$, alors $R_5$ ne soit pas OH ; et que lorsque $R_4$ et $R_5$ sont chacun OR et R est l'hydrogène ou un alkyle en $C_2$-$C_5$, alors $R_6$ et $R_7$ ne soient pas chacun indépendamment un halogène, $NO_2$, CHO ou COOH ;

ou d'un de ses sels acceptables en pharmacie pour la préparation d'un médicament qui inhibe les processus de prolifération cellulaire.

**5.** Utilisation selon la revendication 4, dans laquelle le composé est un composé de formule (Ib), dans laquelle $R_4$ et $R_5$ sont indépendamment OH, $R_6$ est OH et $R_3$ et $R_7$ sont indépendamment l'hydrogène, ou un de ses sels acceptables en pharmacie.

**6.** Utilisation selon la revendication 4, dans laquelle le composé est choisi parmi l'α-hydroxy-(3,4,5-trihydroxybenzylidène)-malononitrile, le 4-formylbenzylidène-malononitrile et leurs sels acceptables en pharmacie.